# EUROPEAN PATENT SPECIFICATION

(11) **EP 0 789 028 B1**
(45) Date of publication and mention of the grant of the patent: **02.05.2002**
(21) Application number: 97101317.2
(22) Date of filing: 29.01.1997
(51) Int. Cl.: C07H 15/248

(54) **A process for the glycosidation of colchicine derivatives and products obtained**
Verfahren zur Glykosylierung von Colchicin-Derivaten und Produkten davon
Procédé pour la glycosidation de dérivés de colchicine et produits obtenus

(30) Priority: 08.02.1996 IT MI960236
(43) Date of publication of application: 13.08.1997
(73) Proprietor: INDENA S.p.A., 20139 Milano (IT)
(72) Inventor: Bombardelli, Ezio, 20139 Milano (IT)
(74) Representative: Minoja, Fabrizio, Dr.

(56) References cited:
- FR-A- 2 112 131
- FR-A- 2 112 710

## Description

The invention relates to novel colchicine or thiocolchicine derivatives obtainable by the process herein described.

Colchicine is known to be a pseudo alkaloid widely used for a long time in therapy for the treatment of gout. Equally diffused in therapy is the use of 3-demethyl-thiocolchicine glucoside, i.e. thiocolchicoside, as decontracting agent and in the inflammation processes of skeletal muscles. 3-Demethylcolchicine glycoside, or colchicoside, is known and studied for pharmacological purposes as well.

FR 2112131 discloses a process for the glycosidation of thiocolchicine which comprises the reaction of acetobromoglucose (or 2,3,4,6-tetra-O-acetyl)-α,D-glycopyranosyl bromide) with 2-demethyl-colchicine or 3-demethyl-thiocolchicine.

The invention relates to compounds having the following general formula I: 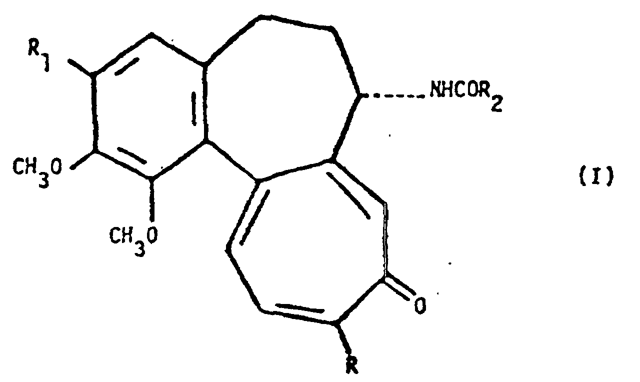
wherein R is a methoxy or thiomethyl group;
R₁ is an optionally protected 6-deoxygalactopyranosyloxy residue;
R₂ is a C₁-C₇ acyl group, preferably acetyl.

The compounds of formula I are obtained by a process comprising the reaction of suitably protected 1-fluorofucose with a compound of formula II: 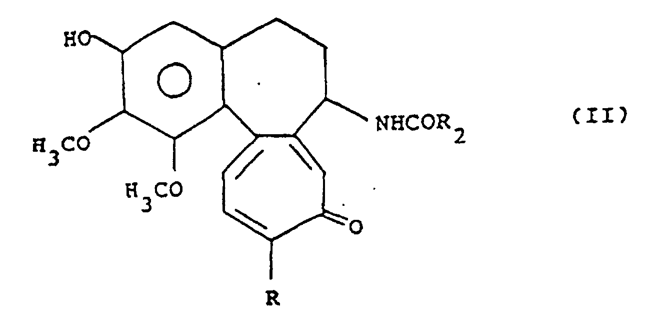 wherein R and R₂ are as defined above, optionally followed by the cleavage of any protecting groups present on the glycide residue.

1-Fluoro fucose derivatives are preferably protected by acetyl groups, i.e. fluoroaceto fucose (1,2,3,4-tetra-0-acetyl-α,L-fucopyranosyl fluoride).

The reaction is preferably carried out in solvents selected from acetonitrile, nitromethane, halogenated hydrocarbons. The use of acetonitrile is particularly preferred. The reaction is carried out for a time ranging from 10 minutes to one hour, at temperatures from 0°C to the solvent's reflux temperature, preferably at room temperature, under inert atmosphere and in the presence of a base. If desired, the step of recovering the protected glycoside product, for example glycoside tetraacetate, can be avoided. The hydrolysis of the protecting groups can be carried out directly on the crude product, to yield the pure product in high yields by direct crystallization. This was not possible with the methods of the prior art, due to the acetobromoglucose strong excesses required.

3-(2'-3,4'-Tri-0-acetyl-L-fucopyranosyl)3-dimethylthiocolchicine is a product having interesting pharmacological properties and it is a specific object of the invention.

The following example illustrates the invention in further detail.

### Example 1

### a) Synthesis of 1,2,3,4-tetra-0-acetyl-α,L-fucopyranose (Rif: G. A. Levvy, A. McAllan, Biochem. J., 80 (1961) 433-439).

L-fucose (5 g) is added in about 20 minutes to a mixture of acetic anhydride (9.2 ml) and pyridine (11 ml), cooling at -5°C. The mixture is left under magnetic stirring, keeping said temperature, for 4 hours and subsequently at 0°C for 2 days, after that it is poured into ice and left under stirring for 4 hours, keeping the temperature at 0°C.

The mixture is extracted with CHCl₃ (3 x 25 ml). The combined organic phases are washed with 10% HCl, (6 x 100 ml), dried over MgSO₄, filtered and the solvent is evaporated off, to obtain a solid crude product (2.05 g) which is crystallized from Et₂O-iPr₂O (1.9 g. 93%). TLC: (AcoEt-Cyclohexane/3:2); the assignment of the absolute configuration of the anomeric carbon is performed by ¹H NMR: in fact, a ³J_{1,2}= 3,4 Hz is observed, corresponding to an axial-equatorial cis coupling. M.p. = 75-76°C.

The resulting fucose tetraacetate is transformed into the corresponding 1-fluoro derivative by treatment with pyridinium polyhydrogen fluoride (2 ml for 1 g of sugar) in anhydrous toluene, analogously to what described above for glucose (R. Noyori et al., Chem. Lett., (1984) 1747-1750), keeping temperature at 5°C for 7 hours.

### b) Synthesis of 3-(2',3',4'-tri-O-acetyl-L-fucopyranosyl)3-demethylthiocolchicine

Operating at room temperature under inert atmosphere, 3-demethylthiocolchicine (88 mg, 0.22 mmol) and 1-fluoroacetofucose (100 mg, 0.33 mmol) are suspended in anhydrous CH₃CN (10 ml). The reaction mixture is added with 1,1,3,3-tetramethylguanidine (83 µl, 0.66 mmol). Following the addition of the base, the reagents are dissolved and the solution is coloured in red. Ether BF₃ (221 µl, 1.76 mmol) is added and the mixture becomes lighter in color. The reaction is left under magnetic stirring, checking by TLC: (MeOH-CH₂Cl₂/0.5:10). The reaction is complete within 30 minutes. A KHCO₃ saturated solution is added and the phases are partitioned; the aqueous phase is extracted with AcOEt. The combined organic phases are washed with a KHSO₄ saturated solution and a NaCl saturated solution, dried over MgSO₄, filtered and the solvent is evaporated off, to obtain a solid crude product (202 mg) which is purified by gravimetric chromatography (MeOH-CH₂Cl₂/0,5:10). The resulting product (135 mg, 0.20 mmol, 90%) is identified by ¹H NMR. Fucoside triacetate is then dissolved in ethanol (2 ml), added with 1N NaOH (1.5 ml) and left under magnetic stirring. The progress of the reaction is checked by TLC: (MeOH-CH₂Cl₂/1:9). The reaction is completed within 1 hour. Fucoside (272 mg, 0.48 mmol, 90%) crystallizes directly from the reaction medium (m.p. 202°C; [α]²²_{D} = -188 (c1, MeOH).

## Claims

1. 3-(2'-3',4'-Tri-O-acetyl-L-fucopyranosyl)-3-demethylthiocolchicine.

2. 3-L-fucopyranosyl-3-demethylthiocolchicine.

## Patentansprüche

1. 3-(2', 3', 4'-Tri-O-acetyl-L-fucopyranosyl)-3-demethylthiocolchicin.

2. 3-L-Fucopyranosyl-3-demethylthiocolchicin.

## Revendications

1. 3-(2'-3',4'-Tri-O-acétyl-L-fucopyranosyl)-3-diméthylthiocolchicine.

2. 3-L-fucopyranosyl-3-diméthylthiocolchicine.
